# EUROPEAN PATENT APPLICATION

(11) **EP 4 755 318 A2**
(43) Date of publication of application: **10.06.2026**
(21) Application number: 26164525.3
(22) Date of filing: 19.04.2021
(51) Int. Cl.: A61B 10/00

(54) **FLUID COLLECTION DEVICES, SYSTEMS, AND METHODS UTILIZING RETENTION COMPONENTS**

(30) Priority: 20.04.2020 US 202063012347 P
(62) Divisional of application: 21724878.0
(71) Applicant: Purewick Corporation, El Cajon, California 92020 (US)
(72) Inventor: KURODA, Melody Mei Hee, Durham, North Carolina 27703 (US); FALLOWS, Eric Alan, Apex, North Carolina 27502 (US); STEVENSON, Florence, Baltimore, Maryland 21230 (US); BIELAWA, Michael, Baltimore, Maryland 21217 (US); MURPHY, Brian, Baltimore, Maryland 21218 (US)
(74) Representative: Hoffmann Eitle

(57) **Abstract**

Embodiments disclosed herein relate to fluid collection devices, systems, and methods utilizing retention components for retaining the fluid collection device in position during use. In an embodiment, a fluid collection device is disclosed. The fluid collection device includes a fluid impermeable barrier at least partially defining an interior chamber and an opening through which the interior chamber is exposed to an external environment. The fluid collection device includes a fluid permeable body positioned at least partially within the interior chamber to extend across at least a portion of the opening and configured to wick fluid away from the opening. The fluid collection device includes one or more retention components for retaining the fluid collection device on labia of a wearer by engaging with one or more of legs, pelvis, or buttocks of the wearer. The fluid collection device includes a conduit extending into the interior chamber.

## Description

### CROSS-REFERENCE TO RELATED APPLICATIONS

This application claims priority to U.S. Provisional Patent Application No. 63/012,347, filed 20 April 2020, the disclosure of which is incorporated herein, in its entirety, by this reference.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, can be used to address some of these circumstances. However, bed pans and urinary catheters have several problems associated therewith. For example, bed pans can be prone to discomfort, pressure ulcers spills, and other hygiene issues. Urinary catheters be can be uncomfortable, painful, and can cause urinary tract infections.

Thus, users and manufacturers of fluid collection devices continue to seek new and improved devices, systems, and methods to collect urine.

### SUMMARY

Embodiments disclosed herein relate to fluid collection devices, systems, and methods utilizing retention components for retaining the fluid collection device in position during use. In an embodiment, a fluid collection device is disclosed. The fluid collection device includes a fluid impermeable barrier at least partially defining an interior chamber and an opening through which the interior chamber is exposed to an external environment. The fluid collection device includes a fluid permeable body positioned at least partially within the interior chamber to extend across at least a portion of the opening and configured to wick fluid away from the opening. The fluid collection device includes one or more retention components for retaining the fluid collection device on labia of a wearer by engaging with one or more of legs, pelvis, or buttocks of the wearer. The fluid collection device includes a conduit extending into the interior chamber.

In an embodiment, a fluid collection system is disclosed. The fluid collection system includes any of the fluid collection devices disclosed herein. The fluid collection system includes a fluid storage container fluidly connected to the fluid collection device via the conduit, the fluid storage container being configured to store fluids therein. The fluid collection system includes a vacuum source fluidly connected to the fluid storage container, the vacuum source being configured to provide vacuum force into the interior chamber via the fluid storage container and the conduit.

In an embodiment, a method to collect fluid is disclosed. The method includes positioning any of the fluid collection devices disclosed herein adjacent to a urethra of a wearer effective to engage one or more retention components for retaining the fluid collection device with one or more of legs, pelvis, or buttocks of the wearer. The method includes receiving fluid from the urethra into the fluid collection device. The method includes removing the fluid from the fluid collection device via the conduit.

### BRIEF DESCRIPTION OF THE DRAWINGS

The drawings illustrate several embodiments of the invention, wherein identical reference numerals refer to identical or similar elements or features in different views or embodiments shown in the drawings.
**FIG. 1** is a schematic diagram of a fluid collection device, according to an embodiment.
**FIG. 2** is an isometric view of a fluid collection device, according to an embodiment.
**FIG. 3** is a cross-sectional view of the fluid collection device of **FIG. 2** taken along the plane A-A, according to an embodiment.
**FIG. 4** is a front view of the device between legs of a wearer, according to an embodiment.
**FIG. 5** is an isometric view of a fluid collection device, according to an embodiment.
**FIG. 6** is an isometric view of a fluid collection device including one or more retention components thereon, according to an embodiment.
**FIG. 7** is an isometric view of the fluid collection device including one or more retention components thereon, according to an embodiment.
**FIG. 8** is an isometric view of a fluid collection device, according to an embodiment.
**FIG. 9** is front view of the fluid collection device of **FIG. 8****,** according to an embodiment.
**FIG. 10** is an isometric view of the fluid collection device positioned on a wearer, according to an embodiment.
**FIG. 11** is an isometric view of a fluid collection device, according to an embodiment.
**FIG. 12** is an isometric view of a fluid collection device, according to an embodiment.
**FIG. 13** is an elevation view of the back of the fluid collection device of **FIG. 12****,** according to an embodiment.
**FIG 14** is an isometric view of a fluid collection device in a first state, according to an embodiment.
**FIG 15** is an isometric view of the fluid collection device of **FIG. 14** in a second state, according to an embodiment.
**FIG 16** is an isometric view of the fluid collection device of **FIG. 14** in the first state disposed on a wearer, according to an embodiment.
**FIG. 17** is a block diagram of a system for collecting fluid, according to an embodiment
**FIG. 18** is a flow diagram of a method for collecting fluid, according to an embodiment.

### DETAILED DESCRIPTION

Embodiments disclosed herein relate to fluid collection devices designed to be placed near the urethra of a wearer and having retention components for using the legs, buttocks, pelvis, or other anatomical structures to maintain the device in position during use to collect fluid, such as urine, from the wearer. Methods of using the fluid collection devices and systems including the fluid collection devices are also disclosed. Example fluid collection devices include a fluid impermeable barrier defining an interior chamber for collecting fluids. The fluid collection devices include a fluid permeable body at least partially disposed within the chamber. The fluid permeable body includes at least one permeable (*e.g*., porous) material for collecting and passing fluids therethrough, such as a wicking material (*e.g.*, foam or spun plastic fibers). The fluid collection body may include a relatively soft fluid permeable membrane disposed over a fluid permeable support material, to interface with the flesh of a wearer. One or more of the fluid impermeable barrier or the fluid permeable body are sized and shaped to fit the wearer near the urethra such as one or more of over the vagina or between the legs of the wearer. The fluid collection devices include one or more retention components for retaining the fluid collection device on labia of a wearer by engaging with one or more of legs, pelvis, buttocks, or other anatomical structures of the wearer.

The devices, systems, and methods disclosed herein provide for secure attachment and retention of the fluid permeable body exposed through the opening of the fluid collection device in position adjacent to (*e.g.,* around, on, over, or in contact with) the urethra or vulva of the wearer.

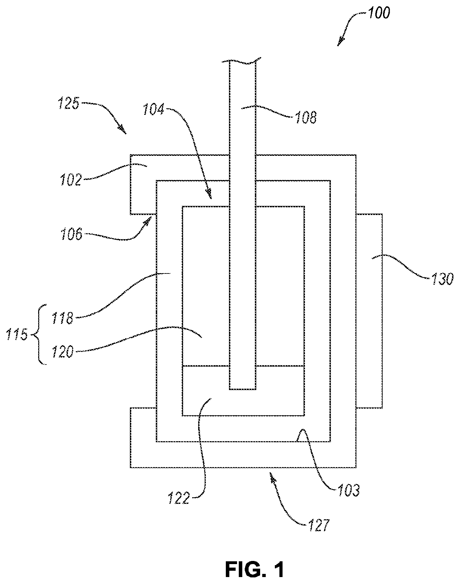

**FIG. 1** is a schematic diagram of a fluid collection device 100, according to an embodiment. The fluid collection device 100 is sized, shaped, and composed to collect urine from the urethra of a wearer. The fluid collection device 100 includes a fluid impermeable barrier 102 defining an interior chamber 104 therein, a fluid permeable body 115 disposed in the interior chamber 104, a conduit 108 disposed in the chamber 104, and one or more retention components 130 for retaining the fluid collection device 100 on the labia of the wearer by engaging with one or more of the legs, pelvis, buttocks, or anatomical structures of the wearer. The fluid permeable body 115 may be exposed to the external environment via the opening 106 in the fluid impermeable barrier 102. During use, the fluid collection device 100 may be positioned over the urethra of the wearer and urine may be received into the fluid collection device 100 by the fluid permeable body 115 via the opening 106. The urine may be removed from the fluid collection device 100 via the conduit 108 disposed within the chamber 104.

The fluid impermeable barrier 102 at least partially defines the interior chamber 104 and opening 106. For example, the inner surface(s) 103 of the fluid impermeable barrier 102 at least partially defines the interior chamber 104 within the fluid collection device 100. The fluid impermeable barrier 102 at least temporarily retains the fluid(s) in the interior chamber 104. The fluid impermeable barrier 102 may be formed of any suitable fluid impermeable material(s), such as a fluid impermeable polymer (*e.g*., silicone, polypropylene, polyethylene, polyethylene terephthalate, thermoplastic elastomer(s), a polycarbonate, etc.), a metal film, natural rubber, another suitable material, or combinations thereof. As such, the fluid impermeable barrier 102 substantially prevents the fluid(s) from passing therethrough. In an example, the fluid impermeable barrier 102 may be air permeable and liquid impermeable. In such an example, the fluid impermeable barrier 102 may be formed of a hydrophobic material that defines a plurality of pores that are air permeable but not liquid permeable. In an example, one or more portions of at least an outer surface of the fluid impermeable barrier 102 may be formed from a soft and/or smooth material, thereby reducing chaffing.

In some examples, the fluid impermeable barrier 102 may be tubular (ignoring the opening), such as substantially cylindrical, oblong, prismatic, flattened tube, or any other extruded shape. The fluid impermeable barrier 102 may be sized and shaped to fit between the legs of a wearer. During use, an outer surface 105 of the fluid impermeable barrier 102 may at least partially contact the wearer, such as the thighs of the wearer.

The opening 106 provides an ingress route for fluids to enter the interior chamber 104. The opening 106 may be defined by the fluid impermeable barrier 102, such as by an inner edge of the fluid impermeable barrier 102. For example, the opening 106 is formed in and extends through the fluid impermeable barrier 102, from the outer surface 105 to the inner surface 103, thereby enabling fluid(s) to enter the chamber 104 from outside of the fluid collection device 100. The opening 106 may be located and shaped to be positioned adjacent to a wearer's urethra while the device is in use. At least a portion of porous material(s) of the fluid permeable body 115 disposed in the chamber 104 may be exposed through the opening 106 to allow fluids to move inwardly into the chamber 104, such as via one or more of permeation, suction, or wicking.

The fluid collection device 100 may be positioned proximate to the urethra and urine may enter the chamber 104 via the opening 106. When in use, the opening 106 may be elongated, extending from a first location below the urethra to a second location above the urethra (*e.g*., at or near the top of the vaginal opening or the pubic region). The opening 106 may exhibit an elongated shape because the space between the legs of a wearer is relatively narrow when the legs of the wearer are closed, thereby only permitting the flow of the fluid(s) along a path that corresponds to the elongated shape of the opening 106 (*e.g.,* longitudinally extending opening).

The opening 106 in the fluid impermeable barrier 102 may exhibit a length that is measured along the longitudinal axis of the fluid collection device 100 that may be at least about 10% of the length of the fluid collection device 100, such as about 25% to about 50%, about 40% to about 60%, about 50% to about 75%, about 65% to about 85%, or about 75% to about 95% of the length of the fluid collection device 100. The opening 106 in the fluid impermeable barrier 102 may exhibit a width that is measured transverse to the longitudinal axis of the fluid collection device 100 and may be at least about 10% of the circumference of the fluid collection device 100, such as about 25% to about 50%, about 40% to about 60%, about 50% to about 75%, about 65% to about 85%, or about 75% to about 100% of the circumference of the fluid collection device 100. The opening 106 may exhibit a width that is greater than 50% of the circumference of the fluid collection device 100 since the vacuum (*e.g*., suction) through the conduit 108 pulls the fluid through the fluid permeable body 115 and into the conduit 108. The opening 106 may be longitudinally oriented (*e.g*., having a major axis parallel to the longitudinal axis of the device 100). In some examples, the opening 106 may be laterally oriented (*e.g*., having a major axis perpendicular to the longitudinal axis of the device 100).

The fluid collection device 100 includes the fluid permeable body 115 disposed in the interior chamber 104. The fluid permeable body 115 may extend across at least a portion (*e.g.,* all) of the opening 106. At least a portion of the fluid permeable body 115 may be exposed to an environment outside of the interior chamber 104 through the opening 106. The fluid permeable body 115 may wick any fluid away from the opening 106, thereby preventing the fluid from escaping the interior chamber 104.

The fluid permeable body 115 includes one or more porous materials. The fluid permeable body 115 may include one or more of a fluid permeable membrane 118 or a fluid permeable support 120. In some examples, the fluid permeable support 120 and the fluid permeable membrane 118 may be made of solely porous material(s), such as a wicking material. At least a portion of the porous material of the fluid permeable body 115 may be a wicking material configured to wick, draw, and/or allow transport any of the bodily fluids away from the opening 106, thereby preventing bodily fluids from escaping the chamber 104. The porous material may not include absorption of the bodily fluids into at least a portion of the porous material. Put another way, substantially no absorption or solubility of the bodily fluids into the porous material may take place after the porous material is exposed to the bodily fluids. While no absorption is desired, the term "substantially no absorption" may allow for nominal amounts of absorption and/or solubility of the bodily fluids into the porous material (*e.g*., absorbency), such as about 30 wt% of the dry weight of the porous material, about 20%, about 10 %, about 7 wt%, about 5 wt%, about 3 wt%, about 2 wt%, about 1 wt%, or about 0.5 wt% of the dry weight of the porous material. In some examples, the porous material may include at least one absorbent or adsorbent material.

The fluid permeable membrane 118 may include any fluid permeable material that may wick the fluid. For example, the fluid permeable membrane 118 may include fabric, such as a gauze (*e.g.,* a silk, linen, or cotton gauze), another soft fabric, or another smooth fabric. The fluid permeable membrane 118 may include spun plastic fibers (*e.g*., nylon), such as a spun plastic mat or bed. Forming the fluid permeable membrane 118 from gauze, soft fabric, and/or smooth fabric may reduce chaffing caused by alternative materials.

The fluid permeable membrane 118 is disposed in the interior chamber 104. The fluid permeable membrane 118 may extend across at least a portion (*e.g*., all) of the opening 106. The fluid permeable membrane 118 may wick fluid inwardly away from the opening 106, thereby preventing fluid from escaping the interior chamber 104.

The fluid permeable body 115 may include the fluid permeable support 120 disposed in the chamber 104. The fluid permeable support 120 is positioned and composed to support the fluid permeable membrane 118 since the fluid permeable membrane 118 may be formed from a foldable, flimsy, or otherwise easily deformable material. For example, the fluid permeable support 120 may be positioned such that the fluid permeable membrane 118 is disposed between at least a portion of the fluid permeable support 120 and the fluid impermeable barrier 102. As such, the fluid permeable support 120 may support and maintain the position of the fluid permeable membrane 118 thereon. The fluid permeable support 120 may include any material that may wick the fluid, such as any of the fluid permeable membrane materials disclosed herein. For example, the fluid permeable support 120 may be formed from any fluid permeable material that is less deformable than the fluid permeable membrane 118, such as any of the materials disclosed herein for the fluid permeable membrane 118, in a more dense or rigid form. In some examples, the fluid permeable support 120 may include a porous polymer (*e.g*., nylon, polyester, polyurethane, polyethylene, polypropylene, etc.) structure, an open cell foam, or spun plastic fibers (*e.g*., nylon fibers). In some examples, the fluid permeable membrane 118 may include gauze and the fluid permeable support may include spun nylon fibers. In some examples, the fluid permeable support 120 may be formed from fabric, felt, gauze, or combinations thereof. In some examples, the fluid permeable support 120 may be formed from a natural material, such as cotton, wool, silk, or combinations thereof. In such examples, the material may have a coating to prevent or limit absorption of fluid into the material, such as a water repellent coating. In some examples, the fluid permeable support 120 may be omitted from the fluid collection device 100. In some examples, the fluid permeable membrane 118 may be optional. For example, the fluid permeable body 115 may include only the fluid permeable support 120.

In some embodiments, the fluid permeable support 120 may have a greater permeability or a greater ability to wick fluids than the fluid permeable membrane 118, such as to move the fluid inwardly from the outer surface of the fluid collection device 100. In some examples, the permeability or the wicking ability of the fluid permeable support 120 and the fluid permeable membrane 118 may be substantially the same.

The fluid permeable membrane 118 and the fluid permeable support 120 may at least substantially completely fill the portions of the chamber 104 that are not occupied by the conduit 108. In another example, the fluid permeable membrane 118 and the fluid permeable support 120 may not substantially completely fill the portions of the chamber 104 that are not occupied by the conduit 108. In such an example, the fluid collection device 100 includes a reservoir 122 in the chamber 104.

The conduit 108 extends into the chamber 104. The conduit 108 may be at least partially disposed in the chamber 104. The conduit 108 (*e.g.,* a drainage tube) includes an inlet and outlet positioned downstream from the inlet. The conduit 108 may extend into the chamber 104 to any point therein. For example, the conduit 108 may be inserted into the chamber 104 at a first end region 125 of the fluid collection device 100 and extend therethrough into the first end region 125 or to the second end region 127. The conduit 108 may extend into the fluid impermeable barrier 102 from the first end region 125 through to the reservoir 122 in the second end region 127 such that the inlet of the conduit 108 is in fluid communication with the reservoir 122. The fluid collected in the reservoir 122 may be removed from the chamber 104 via the conduit 108.

In some examples, the conduit 108 may enter the chamber 104 in the second end region 127 and the inlet of the conduit 108 may be disposed in the second end region 127. The reservoir 122 may be disposed in the second end region 127 in any of the embodiments disclosed herein. The inlet may be spaced from inner surface 103 of the fluid permeable support 120 in the first end region 125. In some examples, the inlet may be disposed at the end of the fluid permeable support 120 in the first end region 125, such as flush with the end of the fluid permeable support 120. In some examples, the inlet may be disposed within the fluid permeable support 120 such between the first end region 125 and the second end region 127.

The conduit 108 may include a flexible material such as plastic tubing (*e.g*., medical tubing). Such plastic tubing may include a thermoplastic elastomer, polyvinyl chloride, ethylene vinyl acetate, polytetrafluoroethylene, etc., tubing. In some examples, the conduit 108 may include silicon or latex. In some examples, the conduit 108 may include one or more portions that are resilient, such as to by having one or more of a diameter or wall thickness that allows the conduit 108 to be flexible. The conduit 108 may be at least partially transparent. In some examples, one or more portions of the conduit 108 may be frosted or opaque (*e.g*., black) to obscure visibility of the fluid(s) therein.

The fluid impermeable barrier 102, the fluid permeable membrane 118 and the fluid permeable support 120 may be sized and shaped to have the conduit 108 at least partially disposed in the chamber 104. For example, at least one of the fluid permeable membrane 118 and the fluid permeable support 120 may be configured to form a space that accommodates the conduit 108. The fluid impermeable barrier 102 may define an aperture sized to receive the conduit 108. The conduit 108 may be disposed in the interior chamber 104 via the aperture. The aperture may be sized and shaped to form an at least substantially fluid tight seal against the conduit 108, thereby substantially preventing the fluid(s) from escaping the interior chamber 104. The fluid collected in the fluid collection device 100 may be removed from the interior chamber 104 via the conduit 108.

The porous material of the fluid permeable body 115 (*e.g.,* fluid permeable membrane 118 and the fluid permeable support 120) may not substantially completely fill the portions of the chamber 104 that are not occupied by the conduit 108. In such examples, the fluid collection device 100 includes the reservoir 122 therein. The reservoir 122 is a substantially unoccupied portion of the chamber 104. The reservoir 122 may be defined between the fluid impermeable barrier 102 and the porous material of the fluid permeable body 115 (*e.g.,* one or both of the fluid permeable membrane 118 and the fluid permeable support 120). The fluid(s) emitted by the wearer may be wicked into the chamber 104 by the porous material of the fluid permeable body 115 and may flow through the fluid permeable membrane 118 and/or fluid permeable support 120 to the reservoir 122. The fluid impermeable barrier 102 may retain the fluid(s) in the reservoir 122. The reservoir 122 may be located in a portion of the fluid collection device expected to be positioned in a gravimetrically low point of the fluid collection device when worn by a person. In such examples, the location of the inlet of the conduit 108 and the reservoir 122 at the gravimetrically low point of the fluid collection device allows the fluids collected in the chamber 104 to drain into the reservoir 122 when the device is positioned on the wearer. For instance, the fluid(s) in the porous material of the fluid permeable body 115 may flow in any direction due to capillary forces. However, the fluid(s) may exhibit a preference to flow in the direction of gravity, especially when at least a portion of the porous material of the fluid permeable body 115 is saturated with the fluid(s). Accordingly, one or more of the inlet or the reservoir 122 may be located in the second end region 127. While depicted in the second end region 127, the reservoir 122 may be located in any portion of the chamber 104 such as the first end region 125. In such examples, the conduit 108 may extend into the reservoir 122, such as through one or more of the porous material of the fluid permeable body 115 or fluid impermeable barrier 102 in the first end region 125.

Other embodiments of fluid impermeable barriers, fluid permeable membranes, fluid permeable supports, cavities, conduits and their shapes and configurations are disclosed in U.S. Patent Application No. 15/612,325 filed on June 2, 2017; U.S. Patent Application No. 15/260,103 filed on September 8, 2016; and U.S. Patent No. 10,226,376 filed on June 1, 2017, the disclosure of each of which is incorporated herein, in its entirety, by this reference.

The fluid collection device 100 includes one or more retention components 130 for retaining the fluid collection device on labia of a wearer by engaging with one or more of legs, pelvis, buttocks, or other anatomical structures of the wearer. The one or more retention components 130 may be one or more additional components attached to one or more of the fluid impermeable barrier 102 or the fluid permeable body 115. While shown as an additional element in **FIG. 1****,** the one or more retention components 130 may be integrated into the construction of one or more of the fluid impermeable barrier 102 or the fluid permeable body 115. Various examples of retention components and fluid collection devices carrying the same are described in more detail below.

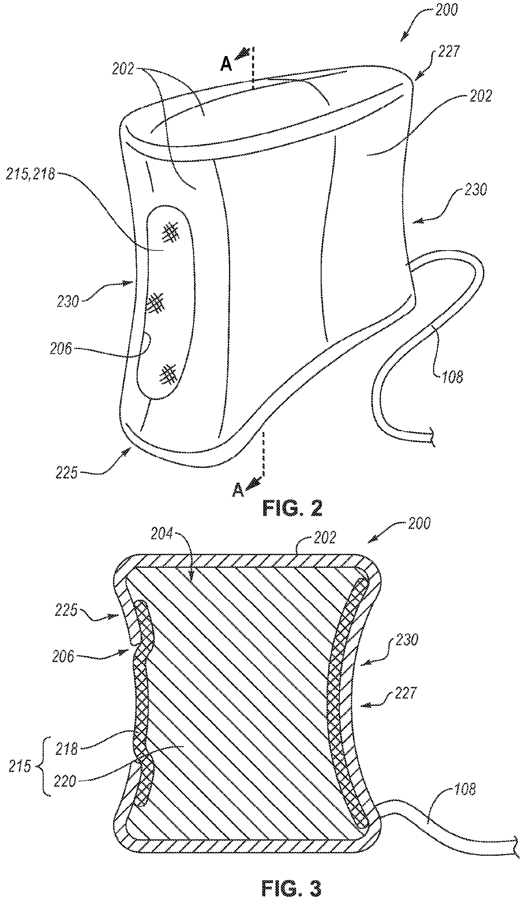

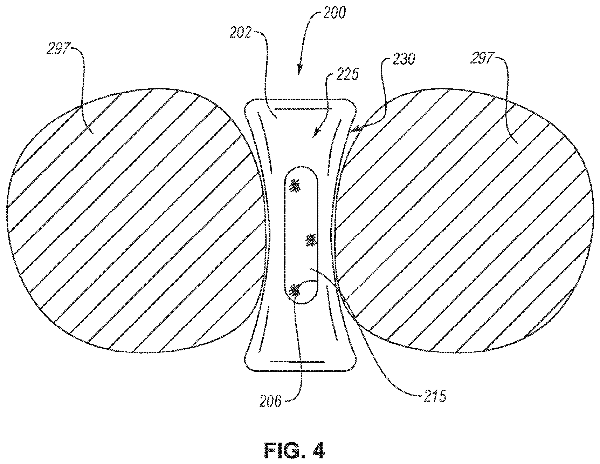

In some examples, one or more portions of the fluid collection device may be shaped to complement, engage with, or be retained in one or more anatomical structures. In such examples, the one or more retention components may include the shape of one or more of the fluid impermeable barrier or fluid permeable body. **FIG. 2** is an isometric view of a fluid collection device 200, according to an embodiment. **FIG. 3** is a cross-sectional view of the fluid collection device 200 of **FIG. 2** taken along the plane A-A, according to an embodiment. **FIG. 4** is a front view of the device 200 between legs 297 of a wearer, according to an embodiment. The fluid collection device 200 includes the fluid impermeable barrier 202, the fluid permeable body 215, both shaped to fit against the inner thighs of a wearer. The one or more retention components 230 include the shape of one or more of the fluid impermeable barrier 202 or fluid permeable body 215. In such examples, the shape includes indentations (*e.g.*, arcuate or angular) in the sides of one or more of the fluid impermeable barrier 202 or fluid permeable body 215 to accommodate surfaces of the inner thighs of a wearer.

The fluid impermeable barrier 202 may be similar or identical to the fluid impermeable barrier 102 in one or more aspects. For example, the fluid impermeable barrier 202 defines the interior chamber 204 (*e.g*., interior cavity or sump of the device) and has an opening 206 therein. The opening 206 is on a front facing surface of the fluid collection device 200. In such examples, the first end region 225 may be the end with the opening 206 thereon, which is the front-facing surface. The size of the fluid impermeable barrier 202 may be relatively large. For example, the fluid impermeable barrier 202 may be sized to fit between, separate, and contour to the shape of the inner thighs of the wearer. The fluid impermeable barrier 202 may have a length (measured from the edge of the barrier in the first end region 225 to the edge of the barrier in the second end region 227) of at least 5 cm, such as 5 cm to 40 cm, 5 cm to 15 cm, 15 cm to 25 cm, less than 40 cm, or less than 25 cm. The fluid impermeable barrier 202 may have a height (*e.g.*, measured transverse to the length along the front of the device 200) of at least 5 cm, such as 5 cm to 25 cm, 5 cm to 15 cm, 15 cm to 25 cm, less than 25 cm, or less than 15 cm. The fluid impermeable barrier 202 may have a width (measured from one side of the front of the device 200 to the other side of the front of the device 200) of at least 3 cm, such as 3 cm to 10 cm, 3 cm to 6 cm, 6 cm to 10 cm, less than 10 cm, or less than 6 cm.

As shown, the fluid impermeable barrier 202 may include a generally polygonal overall shape. The fluid impermeable barrier 202 may include concave sides defining indentations which contour the anatomy of a wearer of the device, such as one or more of the inner thighs, perineal region, or vaginal region of the wearer. For example and as shown in **FIG. 2****,** the sides of the fluid collection device 200 may be concaved with an arcuate or angular indentation to accommodate the thighs of the wearer and the vaginal and perineal regions of the wearer. The indentations forming the one or more retention components 230 may indent at least 1 cm from an outermost portion of the fluid impermeable barrier defining one or more of the length, height, or width thereof. As shown in **FIGS. 2** and **4****,** the sides of the fluid impermeable barrier 202 indent inward to accommodate the shape of the inner thighs of the wearer. As shown in **FIGS. 2** and **3****,** one or both of the front facing side at the first end region 225 or the back facing side at the second end region 227 of the fluid impermeable barrier 202 may have indentations. The indentation on the front facing side at the first end region 225 of the fluid impermeable barrier 202 may be shaped to contour to the perineal and/or vaginal region of the wearer along the sagittal plane.

The fluid permeable body 215 may be similar or identical to the fluid permeable body 115 in one or more aspects. For example, the fluid permeable body 215 may include the fluid permeable membrane 218, the fluid permeable support 220, or both. The fluid permeable membrane 218 may be disposed over at least a portion of the fluid permeable support 220 within the chamber 204. The fluid permeable membrane 218 may be exposed to the external environment via the opening 206, such as to fit between the fluid permeable support 220 and the wearer during use. The opening 206 and fluid permeable membrane 218 are disposed on the front, vaginal-facing portion of the device 200.

Based on the size of the chamber 204, the fluid permeable support 220 may be relatively large to fill the volume of the chamber 204. For example, the majority of the fluid permeable body 215 may be the fluid permeable support 220, such as an open cell foam, spun plastic fibers, or the like. The fluid permeable support 220 and fluid permeable membrane 218 are composed to support the other components of the device 200 but also conform to the wearer's anatomy for added comfort. The fluid permeable body 215 may be relatively rigid internally to prevent collapse of the device 200 with a relatively soft outer portion to prevent bed sores on the interfacing surfaces (*e.g.,* legs 297) of the wearer. The chamber 204 may include structural pillars therein to prevent collapse of the device 200.

The components of the fluid collection device 200 may be sized and shaped to direct fluid collected from the wearer via the opening 206 to a region in the chamber 204 where conduit 108 is located. The conduit 108 may be located at a gravimetrically lowest portion of the device 200 or a point adjacent to the opening 206. While not depicted, the fluid collection device 200 may include a reservoir therein, such as in the second end region 227 to collect fluid therein. Such a reservoir may be similar or identical to the reservoir 122 in one or more aspects. The conduit 108 may be disposed in the reservoir to remove fluid from the device 200.

In some examples (not shown), at least a portion of the device 200 may be removable and replaceable, such as the front portion of the device 200 nearer the first end region 225. The front most portion of the fluid impermeable barrier and the fluid permeable body may be removable from the rest of the fluid impermeable barrier and fluid permeable body such as to be replaced by a clean, unused portion. Additionally, at least a portion of the fluid collection device may be reusable. In such examples, the front portion of the device may be constructed and equipped with the fluid impermeable barrier 202, fluid permeable body 215, and conduit 108 therein to collect fluids and the back portion of the device may be constructed and equipped to mount to the front portion and fit between the wearer's legs to retain the front portion in position during use. The front portion may be removed from the back portion and replaced with a new front portion when soiled by bodily fluids. In such examples, the front and back portions may be separate, with attachments thereon to connect the two portions for use.

In some examples, the back portion of the fluid collection device may include a soft foam which is not intended to be wetted by fluid. Such a back portion may be for comfort or positioning. For example, the back portion near the second end region 227 (that faces away from the groin of the wearer) may be washable and reusable, while the front facing portion of the device near the first end region 225 may be disposable after one use. In such examples, the front facing portion may include the fluid impermeable barrier 202 and the fluid permeable body 215 for interfacing with the wearer. The conduit 108 may be disposed in only the front facing portion. The front facing portion that is replaceable may be about ten percent or more of the total volume of the device 200. At least a portion of the fluid collection device 200 may be washable. For example, the back portion that is separable from the fluid collection device 200 may be washable.

In some examples, at least a portion of the fluid impermeable barrier 202 may be replaced or covered by a soft fabric or layer. For example, the back portion of the fluid collection device 200 may include an outer fabric layer.

In examples, the one or more retention components may include a pressure-sensitive adhesive (PSA) disposed on a front facing surface of the fluid impermeable barrier around at least a portion of the opening. For example, a PSA layer may be included on one or more portions of the outer surface of the fluid impermeable barrier 202 to provide securement between the device 200 and wearer, such as bond to the area near the vulva of the wearer. The PSA layer may be a silicone pressure sensitive adhesive or the like. Such adhesive may be positioned around the opening 206 or on the sides of the fluid impermeable barrier 202. Without an adhesive, the device 200 may include attachments such as straps, flaps, springs, gripping members, or the like for engaging the upper legs or pelvic area to secure the device 200 to the body of the wearer.

As shown in **FIG. 4****,** the front facing side on the first end region 225 of the fluid collection device 200 faces and contacts the wearer's vaginal area to collect and transport urine into chamber 204 via the opening 206. In some examples, the fluid permeable body 215 in the opening 206 may protrude from the fluid impermeable barrier 202 to enable placement on the wearer's labia to promote urine capture. The device 200 may be positioned between the wearer's legs 297 with the fluid permeable body 215 in contact with, or in close proximity to, the wearer's vulva, such as in contact with the labia.

In some examples, the shape of the fluid collection device 200 (*e.g.,* the fluid permeable membrane) may differ from the generally arcuate concaved shape shown in **FIGS. 2-4****.** For example and as described below, the shape of the fluid collection device may be more angular, such as with planar indentations in the sides to contour to the wearer's inner legs and urethral region.

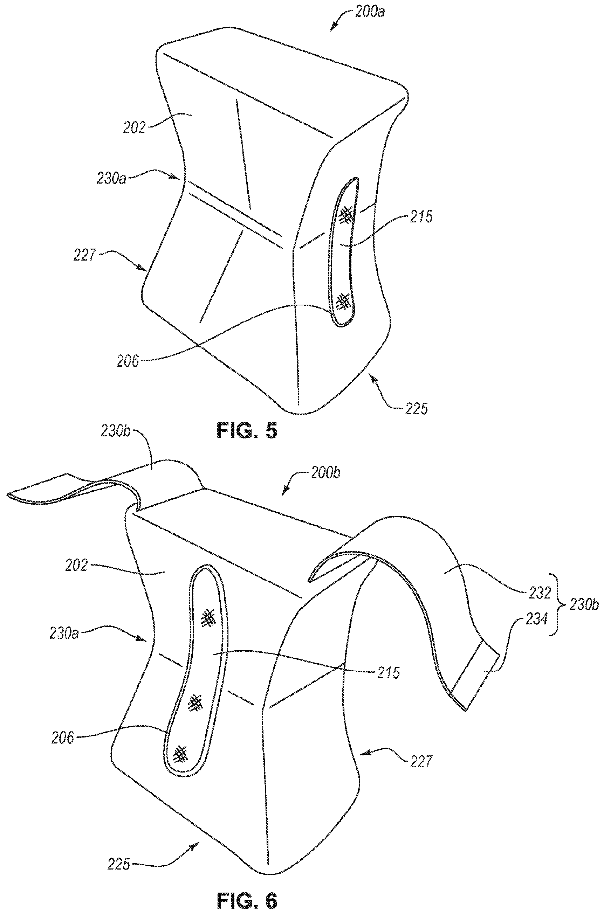

**FIG. 5** is an isometric view of a fluid collection device 200a, according to an embodiment. The fluid collection device 200a is similar or identical to the fluid collection device 200 in one or more aspects. For example, the fluid collection device 200a includes the fluid impermeable barrier 202, the fluid permeable body 215, and one or more retention components 230a. The one or more retention components 230 include the shape of the fluid collection device 200a, such as one or more of the fluid impermeable barrier 202 and the fluid permeable body 215. Indentations formed in the fluid collection device 200a may be shaped to accommodate one or more portions of a wearer as disclosed above with respect to the fluid collection device 200. As shown, the indentations may be angular, such as with planar indentations in the sides of the fluid collection device 200a to contour to the wearer's inner legs and urethral region. The angles forming the indentations may differ on the front side at the first end region 225 and the lateral sides extending from the first end region 225 and the second end region 227. The opening 206 is disposed on the front facing surface to collect fluid from the wearer.

Other positioning or securement approaches may be utilized as one or more retention components, such as adhesive tabs near the pelvic area or straps around waist and/or buttocks area. **FIG. 6** is an isometric view of a fluid collection device 200b including one or more retention components 230b thereon, according to an embodiment. The fluid collection device 200b may be similar or identical to the fluid collection device 200a in one or more aspects. For example, the fluid impermeable barrier 202, the fluid permeable body 215, and one or more retention components 230a. The opening 206 may be disposed on a different surface than the narrower side surface of the fluid collection device 200a (as shown in **FIG. 5****).** For example, the opening 206 may be disposed on the wider side surface as shown in **FIG. 6****.** Accordingly, the distance between the first end region 225 and the second end region 227 may be shorter in fluid collection device 200b than in fluid collection device 200a. In some examples, the opening 206 may be disposed on the same surface as shown in **FIG. 5****.**

The one or more retention components 230b may include straps 232 attached to the fluid impermeable barrier 202. The straps 232 may include tabs, a belt, ties, thongs, wraps, or the like. The straps 232 may be a length selected to at least partially wrap around one or more anatomical structures of the wearer such as the waist, the legs, the pelvis, the buttocks, or the like. The straps 232 may include attachments 234 at the ends thereof. The attachments 234 may be disposed at the end of the straps 232. The attachments 234 may include hook and loop fasteners, adhesive(s), button(s), buckle(s), or the like. Accordingly, the attachments 234 may attach to the wearer or around an anatomical structure of the wearer.

The straps 232 may extend from any end of the device 200b, such as the top end of the device 200b as shown. In some examples, the straps 232 may extend from the front of the device 200b (*e.g.,* end that faces the vaginal region when in use). The straps 232 may attach to the wearer on the thighs, buttocks, pubic region, pelvis, or other portion of the wearer to retain the device 200b in position on the wearer. In some examples, the one or more retention components 230b include adhesive tabs positioned on the fluid impermeable barrier to attach to thighs, buttocks, pubic region, or pelvic area of the wearer.

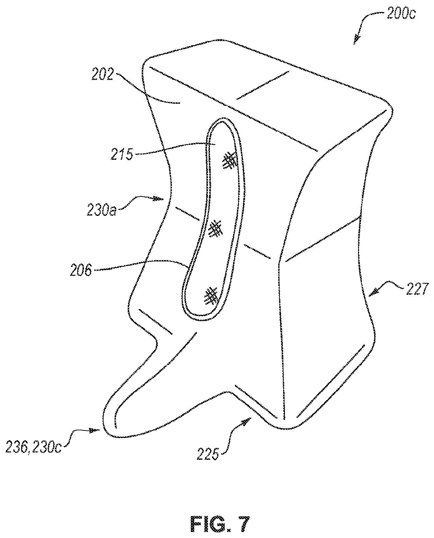

In some examples, the fluid collection device may include one or more protrusions thereon for fitting in an anatomical structure of the wearer. **FIG. 7** is an isometric view of a fluid collection device 200c including one or more retention components 230c thereon, according to an embodiment. The fluid collection device 200c may be similar or identical to the fluid collection device 200b in one or more aspects. For example, the fluid impermeable barrier 202, the fluid permeable body 215, and one or more retention components 230a. The opening 206 may be disposed on the wider side surface as shown in **FIG. 7****.** Accordingly, the distance between the first end region 225 and the second end region 227 may be shorter in fluid collection device 200c than in fluid collection device 200a. The fluid collection device 200c includes the one or more retention components 230c. The one or more retention components 230c may include one or more protrusions 236 for interfacing with the wearer's anatomical features.

The one or more protrusions 236 may be sized, shaped, and positioned on the device 200c to align the opening 206 in position on the wearer and/or to maintain the device 200c (*e.g.,* opening thereof) in selected position on the wearer for use. For example and as shown in **FIG. 7****,** the device 200c may include the protrusion 236 at an end thereof to align the device 200c on the wearer. The one or more protrusions 236 may include a portion of one or more of the fluid impermeable barrier 202 or the fluid permeable body 215. For example, the one or more protrusions 236 may be sized, shaped, and positioned on the fluid impermeable barrier 202 to fit an anatomical feature of the wearer while the opening 206 is located around the urethra of the wearer. The one or more protrusions 236 may include a protrusion sized and shaped to fit within a gluteal cleft of the wearer. As shown, the protrusion 236 may extend from a front facing portion of the fluid collection device 200c below the opening 206.

In some examples, the opening 206 may be disposed on the same surface as shown in **FIG. 5****.** In such examples, the one or more protrusions may extend outwardly from the surface with the opening 206.

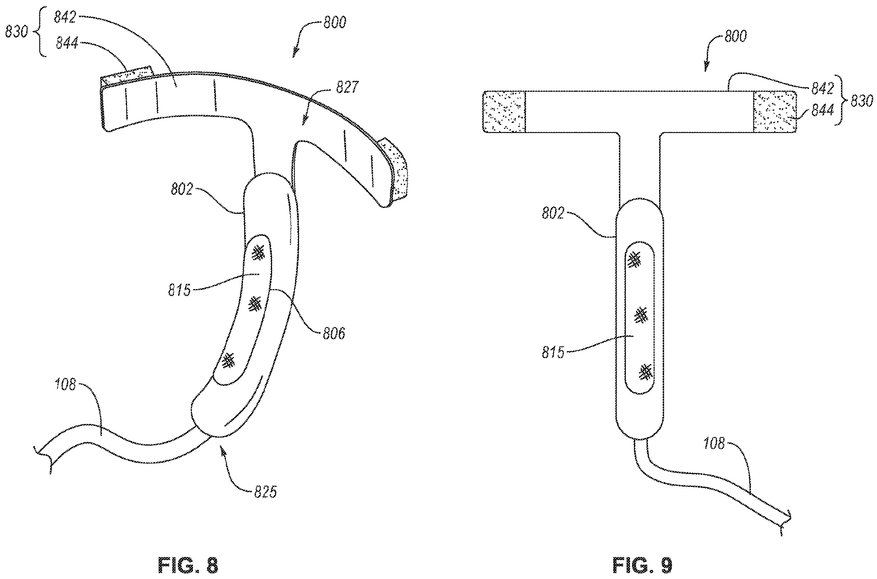

In some examples, the fluid collection devices disclosed herein may include an attachment feature that attaches and/or aligns on the wearer's body, such as the pelvic region. The one or more retention components may include a mounting frame affixed to the fluid impermeable barrier of the fluid collection device. **FIG. 8** is an isometric view of a fluid collection device 800, according to an embodiment. The fluid collection device 800 may be similar or identical to any of the fluid collection devices disclosed herein, such as fluid collection device 100, in one or more aspects. For example, the fluid collection device 800 includes the fluid impermeable barrier 802, the fluid permeable body 815, and the conduit 108. The fluid collection device 800 includes the one or more retention components 830.

As shown, the fluid collection device 800 may be longitudinally oriented, such as substantially cylindrical. The fluid impermeable barrier 802 may be similar or identical to the fluid impermeable barrier 102 in one or more aspects. For example, the fluid impermeable barrier 802 defines a chamber therein. The fluid impermeable barrier 802 includes an opening 806 through which the chamber is in communication with the external environment. The opening 806 is a longitudinally extending hole in the fluid impermeable barrier 802.

The fluid permeable body 815 may be similar or identical to the fluid permeable body 115 in one or more aspects. For example, the fluid permeable body 815 may include the fluid permeable support and the fluid permeable membrane as disclosed herein. The fluid permeable body 815 may be at least partially disposed within the chamber of the fluid collection device 800. The fluid permeable body 815 may not completely fill the chamber. In such examples, the fluid collection device 800 may include a reservoir within the chamber. The reservoir may be defined between the fluid impermeable barrier 802 and the fluid permeable body 815. The reservoir may be located in the first end region 825 or the second end region 827 of the fluid collection device 800.

The conduit 108 may extend into the chamber through the fluid impermeable barrier 802, such as from the first end region 825 to the second end region 827 or only into the first end region 825, such as into the reservoir therein. Fluids (*e.g*., urine) collected in the fluid collection device 800 may be removed via the conduit 108.

The one or more retention components 830 include a mounting frame 842 affixed to the fluid impermeable barrier 802. The mounting frame includes an at least semi-rigid body with a shape that at least partially complements one or more anatomical features of a wearer. The mounting frame 842 may include a polymer, metal, or any other material. The mounting frame 842 may be a semi-rigid frame that extends in a "T" shape, such as from the top (*e.g.*, uppermost longitudinal end) of the device 800. For example, the mounting frame 842 includes a T-shaped at least semi-rigid frame extending from a longitudinal axis of the fluid impermeable barrier 802 and fluid permeable body 815. The "T" shape may include a riser (*e.g*., longitudinal member) extending upward from the fluid impermeable barrier 802 and a cross-member (e.g., lateral member) extending perpendicularly from the top of the riser. The riser and the cross-member may have lengths selected to provide a selected fit against one or more anatomical features of the wearer such as the lower abdomen or waist of the wearer. The riser or the cross-member may be curved to complement the anatomy of the wearer, such as to wrap around at least a portion of the waist, pelvis, or lower abdomen of the wearer.

The mounting frame 842 may include one or more attachment components 844 thereon, such as at the ends of the cross-member. For example, the mounting frame 842 may include adhesives, hook and loop fasteners, weights, or the like to engage with the wearer's skin or clothes to maintain position during use. The one or more attachment components 844 may be positioned on the cross-member such as at the ends of the cross-member. In the case of weights, the passive weight and curvature of the mounting frame 842 and fluid impermeable barrier 802 may allow the device 800 to retain a selected positioning on the wearer such as with the opening on or over the urethra of the wearer. The one or more attachment components 844 may be positioned outward (*e.g*., facing away from the wearer) on the cross-member, such as to attach to the wearer's clothing.

**FIG. 9** is front view of the fluid collection device 800 of **FIG. 8****,** according to an embodiment. As shown in **FIG. 9****,** the attachment components 844 may face inward on the mounting frame 842 in some examples, such as to attach to the wearer or the wearer's clothing.

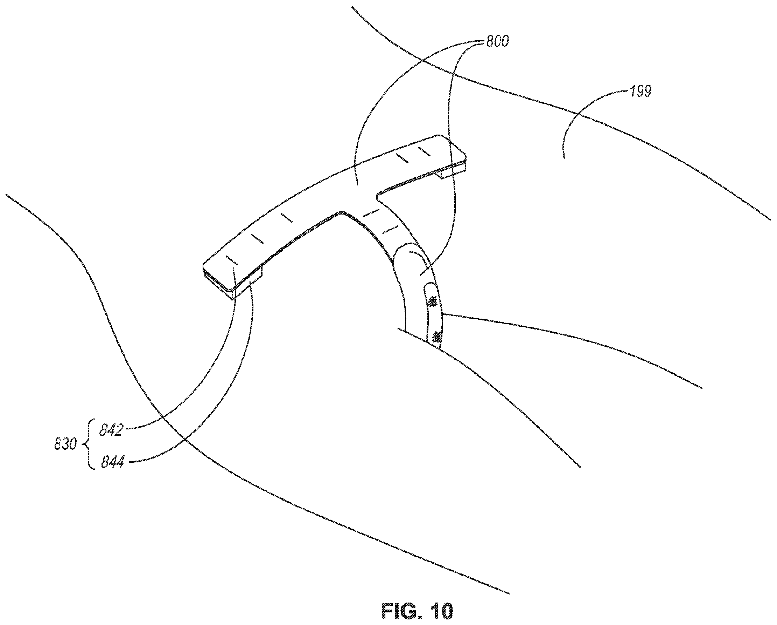

**FIG. 10** is an isometric view of the fluid collection device 800 positioned on a wearer 199, according to an embodiment. As shown, the riser may project upward from the fluid collection device 800 along the wearer's pubic region and the cross-member may extend laterally therefrom to provide an attachment to the wearer's lower abdomen, pelvis, or waist. The one or more attachment components 844 at the ends of the cross-member may attach directly to the wearer. For example, the one or more attachment components 844 may be an adhesive (*e.g.*, hydrogel, PSA, or other skin safe adhesive) which bonds to the wearer's skin, weights for weighing the cross-member down on the wearer's abdomen, hook and loop fasteners for attaching to wearer's clothing, or fasteners (*e.g*., pins, clamps, etc.) for fastening to the wearer's clothing.

The semi-rigid mounting frame 842 may be contoured to hold the device 800 in the correct position on the wearer's anatomy after attachment to the wearer. In some examples, the mounting frame 842 may be fully rigid or may be bendable. In some examples (not shown), straps or a belt may be attached to the mounting frame 842 for additional retention on the wearer.

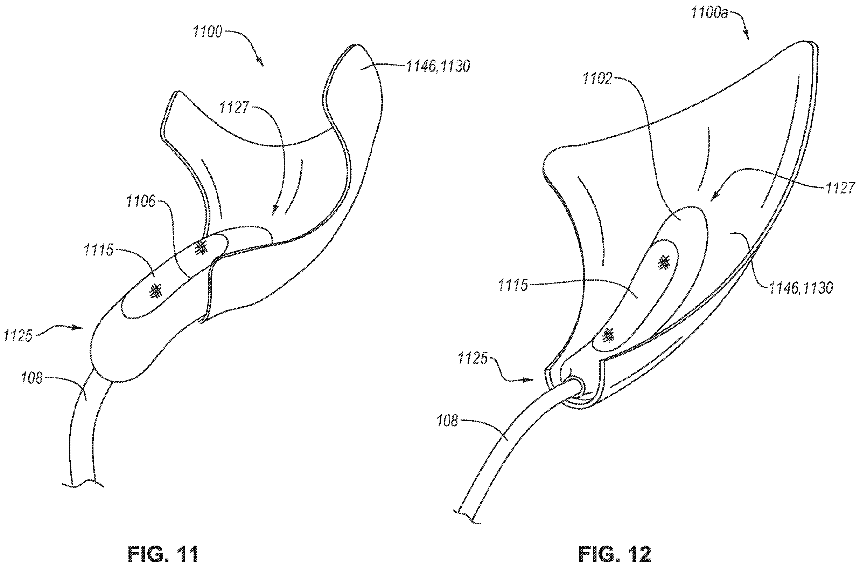

Examples of the fluid collection devices with different mounting frames may be utilized to collect fluids such as urine from female wearers. For example, the fluid collection device may be retained in position by a retention portion that covers the majority of the pubic or pelvic region. **FIG. 11** is an isometric view of a fluid collection device 1100, according to an embodiment. The fluid collection device 1100 may be similar or identical to any of the fluid collection devices disclosed herein, such as fluid collection device 100 or 800, in one or more aspects. For example, the fluid collection device 1100 includes the fluid impermeable barrier 1102, the fluid permeable body 1115, and the conduit 108. The fluid collection device 1100 includes the one or more retention components 1130.

As shown, the fluid collection device 1100 may be longitudinally oriented, such as substantially cylindrical. The fluid impermeable barrier 1102 may be similar or identical to the fluid impermeable barrier 102 or 802 in one or more aspects. For example, the fluid impermeable barrier 1102 defines a chamber therein. The fluid impermeable barrier 1102 includes an opening 1106 through which the chamber is in communication with the external environment. The opening 1106 is a longitudinally extending hole in the fluid impermeable barrier 1102.

The fluid permeable body 1115 may be similar or identical to the fluid permeable body 115 or 815 in one or more aspects. For example, the fluid permeable body 1115 may include the fluid permeable support and the fluid permeable membrane as disclosed herein. The fluid permeable body 1115 may be at least partially disposed within the chamber of the fluid collection device 1100. The fluid permeable body 1115 may not completely fill the chamber. In such examples, the fluid collection device 1100 may include a reservoir within the chamber. The reservoir may be defined between the fluid impermeable barrier 1102 and the fluid permeable body 1115. The reservoir may be located in the first end region 1125 or the second end region 1127 of the fluid collection device 1100.

The conduit 108 may extend into the chamber through the fluid impermeable barrier 1102, such as from the first end region 1125 to the second end region 1127 or only into the first end region 1125, such as into the reservoir therein. Fluids (e.g., urine) collected in the fluid collection device 1100 may be removed via the conduit 108.

The one or more retention components 1130 include a mounting frame 1146 affixed to the fluid impermeable barrier 1102. The mounting frame 1146 includes a body with a shape that at least partially complements one or more anatomical features of a wearer. The mounting frame 1146 may include a polymer, rubber, fabric, silicone, or any other material. The mounting frame 1146 may be at least semi-rigid. The mounting frame 1146 may be at least partially flexible to accommodate variations in the shape of wearers and movement of the wearer during use. The mounting frame 1146 of the one or more retention components 1130 may extend outwardly and upward from the fluid impermeable barrier 1102, such as from the second end region 1127 thereof. The mounting frame 1146 may be sized, shaped, and disposed on the fluid impermeable barrier 1102 to provide a securement on the wearer which locates the opening over the urethra (*e.g*., labia or vulva) of the wearer. The mounting frame 1146 may be disposed on the fluid impermeable barrier 1102 in a position to allow the fluid permeable body 1115 in the opening to be disposed in the region of the urethra of the user (*e.g.,* near the labia) when attached to the wearer. In an example, the mounting frame 1146 may be a silicone body (*e.g.,* sheet or plate) that is affixed to or integrally formed with the fluid impermeable barrier, where the silicone body is at least partially flexible. The mounting frame 1146 may be attached to the fluid impermeable barrier 1102 on a side thereof opposite the opening 1106 or around the sides of the opening 1106.

The mounting frame 1146 may have a lengths, width, and shape selected to provide cover and fit against one or more anatomical features of the wearer, such as the lower abdomen or pubic region of the wearer. The mounting frame 1146 may be substantially triangular, with a narrowest point attaching to the fluid impermeable barrier 1102 and expanding laterally as the mounting frame 1146 extends longitudinally way from the fluid impermeable barrier 1102. In such examples, the mounting frame 1146 may complement the shape of a wearers pubic region or lower abdomen. The mounting frame 1146 may be curved to complement the anatomy of the wearer, such as to wrap around at least a portion of the pubic region, waist, pelvis, or lower abdomen of the wearer.

The mounting frame 1146 may be retained in position on the wearer by frictional engagement against the skin or clothing of the wearer, such as by mating the relative large surface area of the mounting frame 1146 against the wearer. In some examples, one or more retention components 1130 (mounting frame 1146) may include one or more attachment components such as an adhesive, hook and loop fasteners, attachments, weights, or the like thereon. The one or more attachment components may engage with the wearer or their clothing to maintain positioning of the fluid permeable body 1115 in the opening over the urethra of the wearer. In such examples, the one or more attachment components may be positioned along the top edge of the mounting frame 1146, such as at the lateral ends thereof on the wearer facing side or the opposite side thereof. The one or more attachment components may be disposed around an outermost boundary of the mounting frame 1146.

In some examples, the mounting frame 1146 may be attached to the fluid impermeable barrier on a different point than in the second end region 1127. **FIG. 12** is an isometric view of a fluid collection device 1100a, according to an embodiment. The fluid collection device 1100a is similar or identical to the fluid collection device 1100 in one or more aspects. For example, the fluid collection device includes the fluid impermeable barrier 1102, the fluid permeable body 1115, and the one or more retention components 1130 including the mounting frame 1146. The mounting frame 1146 may be mounted to the fluid impermeable barrier 1102 in a different location than in the fluid collection device 1100 **(****FIG. 11****).** For example, the mounting frame 1146 may be mounted to the fluid impermeable barrier 1102 at the first end region 1125 and extend longitudinally past the second end region 1127. In such examples, the fluid impermeable barrier 1102 and the fluid permeable body 1115 may be positioned more centrally within the mounting frame 1146 than the example shown in **FIG. 11****.** Such a configuration may provide a fit which positions the opening 1106 over the urethra of the wearer while the mounting frame fits against and complements the shape of the pubic region or lower abdominal region of the wearer.

In some examples, the fluid impermeable barrier 1102 may be sandwiched between the mounting frame 1146 and a fluid permeable membrane material on at least a portion of the front (*e.g*., wearer facing) surface of the mounting frame 1146. In such examples, the fluid permeable membrane material on the mounting frame 1146 may be similar or identical to any of the fluid permeable membranes disclosed herein, such as a gauze or the like.

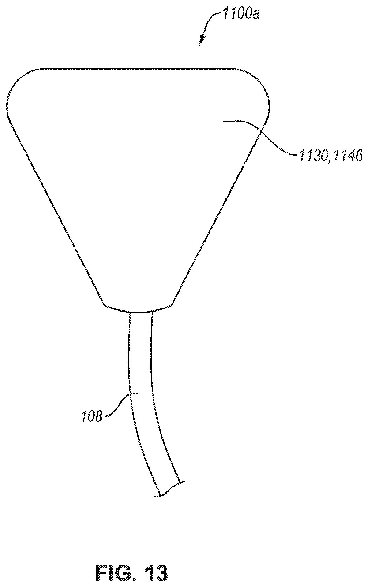

**FIG. 13** is an elevation view of the back of the fluid collection device 1100a of **FIG. 12****,** according to an embodiment. As shown, the back of the mounting frame 1146 of the fluid collection device 1100a may completely cover the fluid impermeable barrier 1102. Accordingly, the fluid impermeable barrier 1102 and the fluid permeable body 1115 may not be visible from the back side of the fluid collection device 1100a.

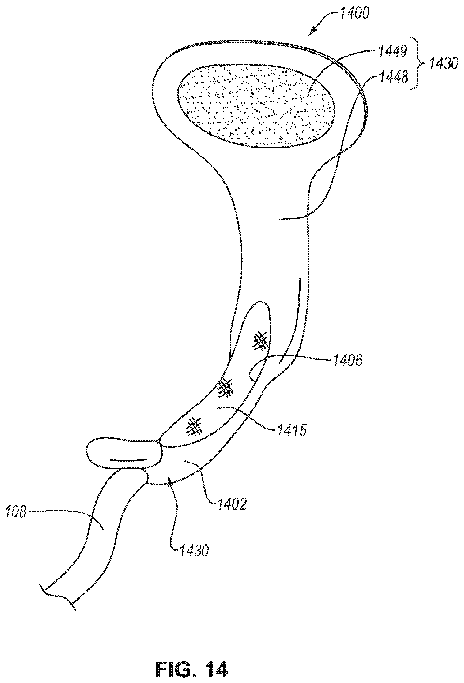

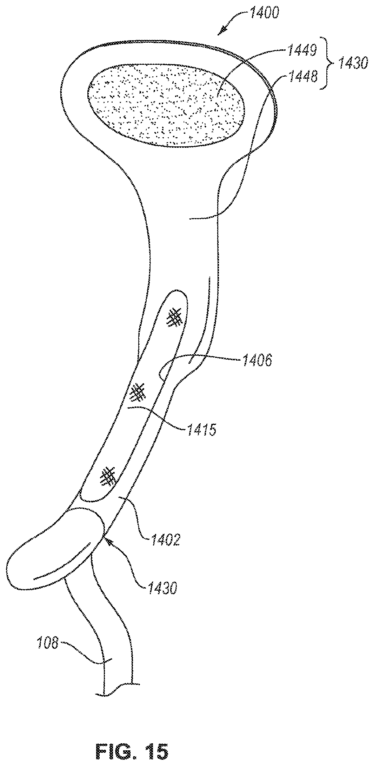

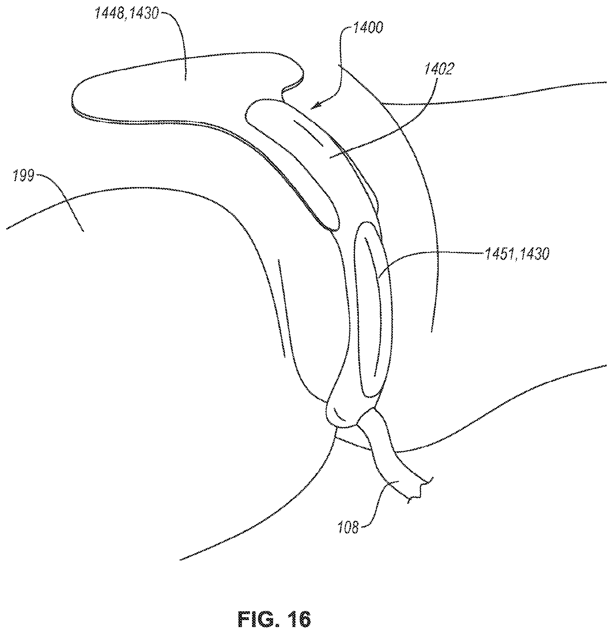

In some examples, the one or more retention components may include a spring member. For example, the fluid collection device may be maintained in position on the wearer by attachment to the pelvis at the pubic region and pressure from a spring member. **FIG 14** is an isometric view of a fluid collection device 1400 in a first state, according to an embodiment. **FIG 15** is an isometric view of the fluid collection device 1400 of **FIG. 14** in a second state, according to an embodiment. **FIG 16** is an isometric view of the fluid collection device 1400 of **FIG. 14** in the first state disposed on a wearer 199, according to an embodiment. The fluid collection device 1400 may be similar or identical to any of the fluid collection devices disclosed herein, such as fluid collection device 100, 800, or 1100, in one or more aspects. For example, the fluid collection device 1400 includes the fluid impermeable barrier 1402, the fluid permeable body 1415, and the conduit 108. The fluid collection device 1400 includes the one or more retention components 1430.

As shown, the fluid collection device 1400 may be longitudinally oriented, such as substantially cylindrical in one or more portions thereof. The cylindrical body of the device 1400 may be flexible to contour to the anatomy of the wearer. The fluid impermeable barrier 1402 may be similar or identical to the fluid impermeable barrier 102, 802, or 1102 in one or more aspects. For example, the fluid impermeable barrier 1402 defines a chamber therein. The fluid impermeable barrier 1402 includes an opening 1406 through which the chamber is in communication with the external environment. The opening 1406 is a longitudinally extending hole in the fluid impermeable barrier 1402.

The fluid permeable body 1415 may be similar or identical to the fluid permeable body 115, 815, or 1115 in one or more aspects. For example, the fluid permeable body 1415 may include the fluid permeable support and the fluid permeable membrane as disclosed herein. The fluid permeable body 1415 may be at least partially disposed within the chamber of the fluid collection device 1400. The fluid permeable body 1415 may not completely fill the chamber. In such examples, the fluid collection device 1400 may include a reservoir within the chamber. The reservoir may be defined between the fluid impermeable barrier 1402 and the fluid permeable body 1415. The reservoir may be located in the first end region 1425 or the second end region 1427 of the fluid collection device 1400, such as in a portion thereof expected to be at a gravimetrically low point of the fluid collection device 1400 when in use.

The conduit 108 may extend into the chamber through the fluid impermeable barrier 1402, such as from the first end region 1425 to the second end region 1427 or only into the first end region 1425, such as into the reservoir therein. Fluids (e.g., urine) collected in the fluid collection device 1400 may be removed via the conduit 108.

The one or more retention components 1430 include a mounting frame 1448 affixed to or integrally formed with the fluid impermeable barrier 1402. The mounting frame 1448 may be attached to the fluid impermeable barrier 1402 on a side thereof opposite the opening 1406 or around the sides of the opening 1406. The mounting frame 1448 may be disposed on the fluid impermeable barrier 1402 in a position to allow the fluid permeable body 1415 in the opening 1406 to be disposed in the region of the urethra of the wearer (*e.g.*, near the labia) when attached to the wearer. The mounting frame 1448 includes a body with a shape that at least partially complements one or more anatomical features of a wearer. The mounting frame 1448 may have a length, width, and shape selected to provide cover and fit against one or more anatomical features of the wearer. In such examples, the mounting frame 1448 may complement the shape of a wearer's pubic region or lower abdomen. The mounting frame 1448 may be curved to complement the anatomy of the wearer, such as to wrap around at least a portion of the pubic region, waist, pelvis, or lower abdomen of the wearer. The mounting frame 1448 may be at least semi-rigid. The mounting frame 1448 includes a material therein selected to create an inward spring force on the mounting frame 1448, such as a force which tends for bias the ends of the fluid collection device 1400 inwardly towards each other. For example, the mounting frame 1448 may include a polymer, a metal, a rubber, silicone, or the like.

The mounting frame 1448 may be retained in position on the wearer by frictional engagement against the skin or clothing of the wearer, such as by mating the relative large surface area of the mounting frame 1448 against the wearer. In some examples, one or more retention components 1430 (*e.g*., mounting frame 1448) may include one or more attachment components 1449 such as an adhesive, hook and loop fasteners, attachments, weights, or the like thereon. The one or more attachment components 1449 may engage with the wearer or their clothing to maintain positioning of the fluid permeable body 1415 in the opening over the urethra of the wearer. In such examples, the one or more attachment components 1449 may be positioned along the top edge of the mounting frame 1448, such as in the lateral portion thereof, on the wearer facing side or the opposite side thereof. In some examples, the one or more attachment components 1449 may be disposed around an outermost boundary of the mounting frame 1448.

The mounting frame 1448 may be at least partially flexible to accommodate variations in the shape of wearers and movement of the wearer during use. The mounting frame 1448 may be sized and shaped to provide inward force when forced open. One or more of the mounting frame 1448, the fluid impermeable barrier 1402, and the fluid permeable body 1415 may individually or collectively form a spring member. In some examples, the mounting frame 1448 is at least a portion of a spring member having a size and shape to provide inward force when the spring member is forced open. The spring member may provide an inward bias to clamp inwardly against one or more anatomical structures of the wearer, such as the perineum, labia, pubic region, or lower abdomen of the wearer. Such clamping force may be applied along the sagittal plane of the wearer. The spring force may be provided by selectively controlling the dimensions of the device 1400 such that the device 1400 must be slightly opened to be positioned on the wearer.

In some examples, the spring member may be sized, shaped, and composed to be a bi-state spring, such as with an clamping state and open state.

The mounting frame 1448 may include a longitudinal portion which extends longitudinally away from the second end region 1427. The longitudinal portion may be arcuate to complement one or more anatomical structures of the wearer along the sagittal plane and provide an inward spring force. The mounting frame 1448 may include a lateral portion that extends substantially perpendicularly to the longitudinal portion such as perpendicularly from the end of the longitudinal portion. The lateral portion may extend perpendicularly to the longitudinal axis of the fluid impermeable barrier. The lateral member may have an arcuate shape that contours a waist region of the wearer.

As shown in **FIG. 16****,** in some examples the spring member may include a separate longitudinal member 1451 that extends along a longitudinal axis of one or more of the fluid impermeable barrier 1402 and the mounting frame 1448. In such examples, the longitudinal member 1451 may have an arcuate shape that contours a pubic region and perineal region of the wearer. The longitudinal member 1451 may be disposed on or at least partially within at least a portion of the fluid impermeable barrier 1402. For example and as shown, the longitudinal member 1451 may extend along the back of the fluid impermeable barrier 1402 (opposite the opening 1406).

In some examples, the spring member may extend beyond a first end of the fluid impermeable barrier 1402 and a second end of the fluid impermeable barrier 1402. For example, the spring member may include the mounting frame 1448 at the second end region 1427 and an extension extending from the first end region 1425. The extension may be a portion of a spring member (*e.g*., rubber or silicone protrusion) extending longitudinally from the first end region 1425 of the fluid impermeable barrier 1402. The extension may be formed of a material similar or identical to the fluid impermeable barrier 1402. In some examples, the extension may include the longitudinal member (*e.g*., metal or polymer member) therein. In some examples, the extension may include a reservoir. In some examples, the conduit 108 may be disposed in the extension.

As shown in **FIGS. 14** and **16****,** the first state of the one or more retention components 1430 (*e.g.,* spring member) may be in a clamped or most inwardly biased state. In order to position the fluid collection device 1400 on the wearer, the one or more retention components 1430 (*e.g.,* spring member) may be forced open to place the retention components in an open state. **FIG. 15** depicts the fluid collection device 1400 in the second open state (*e.g*., have a larger arc than the first closed or clamped state).

As shown in **FIG. 16****,** the inward clamping force provided by the one or more retention components 1430 (*e.g*., adhesive and spring member) may allow the fluid collection device 1400 to be positioned and retained with the opening over the urethra of the wearer, such as on the labia. The first end region of the fluid collection device 1400 may be positioned in or near the perineum or anus of the wearer and the second end of the fluid collection device 1400 may be disposed on the lower abdomen or pubic region of the wearer. The anchor provided by the attachment by the adhesive may provide additional leverage to the spring member to clamp onto the anatomy of the wearer.

Any of the retention components of any of the fluid collection devices disclosed herein may be used with any of the other fluid collection devices disclosed herein, such as in additional to or alternatively to the fluid collection devices described therewith.

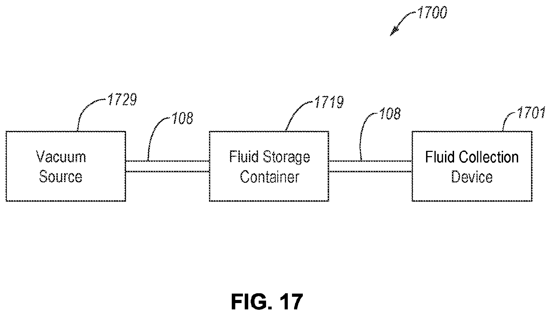

Any of the fluid collection devices disclosed herein may be used in systems for collecting fluids such as urine and vaginal discharge from a wearer. **FIG. 17** is a block diagram of a system 1700 for collecting fluid, according to an embodiment. The system 1700 includes a fluid collection device 1701, a fluid storage container 1719, and a vacuum source 1729. The fluid collection device 1701, the fluid storage container 1719, and the vacuum source 1729 may be fluidly coupled to each other via one or more conduits 108. For example, fluid collection device 1701 may be operably coupled (*e.g*., fluidly connected) to one or more of the fluid storage container 1719 or the vacuum source 1729 via the conduits 108.

The fluid collection device 1701 may be similar or identical to any of the fluid collection devices disclosed herein. For example, the fluid collection device 1701 may include a fluid impermeable barrier, a fluid permeable body, and one or more retention components, as disclosed herein. The fluid collection device may include any of the one or more retention components disclosed herein. The fluid collection device 1701 may include the conduit 108 including an inlet and an outlet as disclosed herein. The outlet may be fluidly coupled to the fluid storage container 1719 and the inlet may be positioned in the fluid collection device 1701 such as in a portion of the chamber therein selected to be at a gravimetrically low point of the fluid collection device 1701 when worn by a user (*e.g.,* reservoir).

The conduit 108 is coupled to and at least partially extends between one or more of the fluid storage container 1719 and the vacuum source 1729. Accordingly, the vacuum source 1729 may be fluidly connected to the fluid storage container 1719 via the conduit 108. In an example, the conduit 108 is directly connected to the vacuum source 1729. In some examples, the conduit 108 may be indirectly connected to at least one of the fluid storage container 1719 and the vacuum source 1729. In some examples, the conduit 108 may be secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063, the disclosures of which are all incorporated herein by reference in their entirety.

Fluid (*e.g*., urine or other bodily fluids) collected in the fluid collection device 1701 may be removed from the fluid collection device 1701 via the conduit 108. Vacuum or suction force may be applied to remove fluid from the fluid collection device via the conduit either directly or indirectly. The vacuum force may be applied indirectly via the fluid storage container 1719. For example, the second open end of the conduit 108 may be disposed within the fluid storage container 1719 and an additional conduit 108 may extend from the fluid storage container 1719 to the vacuum source 1729. Accordingly, the vacuum source 1729 may indirectly apply vacuum or suction force into the fluid collection device 1701 (*e.g*., chamber therein) via the fluid storage container 1719 and conduit 108. As the fluid is drained from the chamber of the fluid collection device 1701, the fluid may travel through the first section of conduit 108 to the fluid storage container 1719 where it may be retained. The fluid storage container 1719 is constructed to store fluids therein. The fluid storage container 1719 may be a substantially rigid container, such as a jar, a canister, or the like. The fluid storage container may include a bag in some examples.

The vacuum source 1729 may include one or more of a vacuum pump, a wall-mounted vacuum line, or a hand pump. For example, the vacuum source 1729 may include one or more of a manual vacuum pump, and electric vacuum pump, a diaphragm pump, a centrifugal pump, a displacement pump, a magnetically driven pump, a peristaltic pump, or any pump configured to produce a vacuum. In examples, the vacuum source 1729 may be a portable vacuum source powered by one or more of a power cord (*e.g*., connected to a power socket), one or more batteries, or even manual power (*e.g.,* a hand operated vacuum pump).

Any of the fluid collection devices or systems disclosed herein may be utilized the collect one or more fluids from a wearer of the fluid collection device.

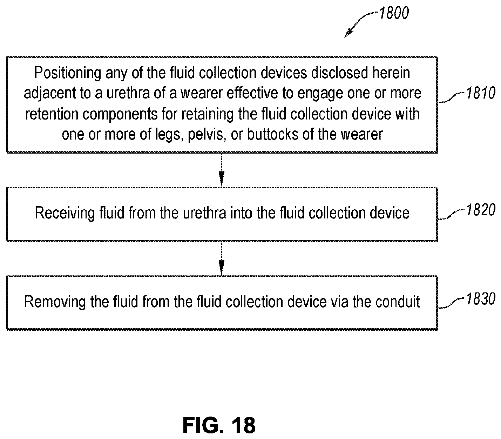

**FIG. 18** is a flow diagram of a method 1800 for collecting fluid, according to an embodiment. The method 1800 includes a block 1810 of positioning any of the fluid collection devices disclosed herein adjacent to a urethra of a wearer effective to engage one or more retention components for retaining the fluid collection device with one or more of legs, pelvis, or buttocks of the wearer; a block 1820 of receiving fluid from the urethra into the fluid collection device; and a block 1830 of removing the fluid from the fluid collection device via the conduit. Any the blocks 1810, 1820, or 1830 of the method 1800 may be performed in different orders, split into multiple acts, modified, supplemented, or combined. For example, the blocks 1820 and 1830 may be combined into a single block. In some examples, one or more of the blocks 1810-1830 of the method 1800 may be omitted.

Block 1810 of positioning any of the fluid collection devices disclosed herein adjacent to a urethra of a wearer effective to engage one or more retention components for retaining the fluid collection device with one or more of legs, pelvis, or buttocks of the wearer may include utilizing any of the fluid collection devices disclosed herein. Positioning any of the fluid collection devices disclosed herein adjacent to a urethra of a wearer may include positioning the opening on, around or over the labia or vulva of the wearer. Positioning any of the fluid collection devices disclosed herein adjacent to a urethra of a wearer effective to engage one or more retention components for retaining the fluid collection device with one or more of legs, pelvis, or buttocks of the wearer may include using any of the retention components disclosed herein to engage with (*e.g*., retain position relative to) one or more anatomical structures of the wearer.

In some examples, the one or more retention components include the shape of the fluid impermeable barrier and fluid permeable body as disclosed herein. For example, the shape may include indentations in the sides of the fluid impermeable barrier and fluid permeable body to accommodate surfaces of inner thighs of the wearer and the front facing surface includes the opening. In such examples, positioning any of the fluid collection devices disclosed herein adjacent to a urethra of a wearer effective to engage one or more retention components for retaining the fluid collection device with one or more of legs, pelvis, or buttocks of the wearer may include disposing the fluid collection device between the legs of the wearer such that the indentations are disposed against the inner thighs of the wearer and the opening is disposed over the urethra.

In some examples, the one or more retention components may include a pressure sensitive adhesive disposed on the front facing surface around at least a portion of the opening of the fluid collection device. In such examples, positioning any of the fluid collection devices disclosed herein adjacent to a urethra of a wearer effective to engage one or more retention components for retaining the fluid collection device with one or more of legs, pelvis, or buttocks of the wearer may include placing the pressure sensitive adhesive in contact with one or more of a pubic region, perineal region, or labia of the wearer.

In some examples, the one or more retention components may include one or more of straps, flaps, springs, or gripping members attached to the fluid impermeable barrier for engaging upper legs or pelvic area to secure the device to the wearer. In such examples, positioning any of the fluid collection devices disclosed herein adjacent to a urethra of a wearer effective to engage one or more retention components for retaining the fluid collection device with one or more of legs, pelvis, or buttocks of the wearer may include engaging one or more of the straps, flaps, springs, or gripping members with one or more of the upper legs or pelvic area of the wearer. Such engagement may include wrapping, strapping, belting, etc. the straps, flaps, springs, tabs, gripping members or the like around one or more the upper legs, pelvic region, waist, buttocks, or other anatomical features of the wearer.

In some examples, the one or more retention components may include adhesive tabs positioned on the fluid impermeable barrier to attach to thighs, buttocks, pubic region, or pelvic area of the wearer. In such examples, positioning any of the fluid collection devices disclosed herein adjacent to a urethra of a wearer effective to engage one or more retention components for retaining the fluid collection device with one or more of legs, pelvis, or buttocks of the wearer may include placing the adhesive of the adhesive tabs in contact with the thighs, buttocks, pubic region, or pelvic area of the wearer. The adhesive tabs may be similar or identical to any of the adhesive bodies disclosed herein.

In some examples, the one or more retention components may include one or more protrusions sized, shaped, and positioned on the fluid impermeable barrier to fit an anatomical feature of the wearer. In such examples, positioning any of the fluid collection devices disclosed herein adjacent to a urethra of a wearer effective to engage one or more retention components for retaining the fluid collection device with one or more of legs, pelvis, or buttocks of the wearer may include disposing the one or more protrusions into, between, or on the anatomical feature (structure) of the wearer, such as in the gluteal cleft of the wearer.

In some examples, the one or more retention components may include a mounting frame affixed to the fluid impermeable barrier, such as any of the fluid impermeable barriers disclosed herein. The mounting frame may have an at least semi-rigid body with a shape that at least partially complements one or more anatomical features of a wearer. In such examples, positioning any of the fluid collection devices disclosed herein adjacent to a urethra of a wearer effective to engage one or more retention components for retaining the fluid collection device with one or more of legs, pelvis, or buttocks of the wearer may include positioning the mounting frame around the one or more anatomical features of the wearer, such as on or around the pubic region of the wearer, the pelvis of the wearer, or the waist of the wearer. In some examples, the mounting frame includes one or more attachment components thereon. In such examples, positioning the mounting frame around the one or more anatomical features of the wearer may include attaching the one or more attachment components to the wearer or clothing of the wearer.

In some examples, the mounting frame may be sized, shaped, and composed to form at least part of a spring member having a size and shape to provide inward force when the spring member is forced open. In such examples, positioning any of the fluid collection devices disclosed herein adjacent to a urethra of a wearer effective to engage one or more retention components for retaining the fluid collection device with one or more of legs, pelvis, or buttocks of the wearer may include opening the mounting frame, positioning the mounting frame around at least a portion of one or more of the legs, pelvis, perineal region, pubic region, lower abdominal region, or buttocks of the wearer, and subsequently, releasing the mounting frame. Releasing the mounting frame allows the mounting frame to clamp onto one or more anatomical structures of the wearer to clamp onto the wearer.

Any of combination of any of the one or more retention features disclosed herein may be utilized to engage with a portion of one or more of the legs, pelvis, perineal region, pubic region, lower abdominal region, or buttocks of the wearer to retain the fluid collection device in position during use.

Block 1820 of receiving fluid from the urethra into the fluid collection device may include receiving the fluid into fluid permeable body, such as via the opening. Receiving fluid from the urethra into the fluid collection device may include wicking or otherwise allowing the fluid to flow into the chamber of the fluid collection device via the fluid permeably body (*e.g.,* fluid permeable membrane and fluid permeable support). Receiving fluid from the urethra into the fluid collection device may include may include flowing the fluid towards a portion of the chamber of the fluid collection device that is fluidly coupled to one or more of a gravimetrically low portion of the fluid chamber, an inlet of a conduit, or a reservoir.

Block 1830 of removing the fluid from the fluid collection device via the conduit may include removing at least some of the fluid from the chamber of the fluid collection device. Removing the fluid from the fluid collection device via the conduit may include removing at least some of the fluid from the fluid permeable body of the fluid collection device. Removing the fluid from the fluid collection device via the conduit may include applying a vacuum in the chamber, such as via any of the vacuum sources disclosed herein. In such examples, the removing may include activating the vacuum source. Removing the fluid from the fluid collection device via the conduit may include removing the fluid into a fluid storage container.

The method 1800 may include collecting the fluid(s) that are removed from the fluid collection device, such as in the fluid storage container. The method may include one or more of testing or quantifying the amount of fluid removed.

The fluid collection devices, systems, and methods disclosed herein provide for secure attachment of fluid collection devices to collect fluids emitted from a wearer, which prevents leakage and soiling of the wearer's clothing, bedding, and care takers.

As used herein, the term "about" or "substantially" refers to an allowable variance of the term modified by "about" by ±10% or ±5%. Further, the terms "less than," "or less," "greater than", "more than," or "or more" include as an endpoint, the value that is modified by the terms "less than," "or less," "greater than," "more than," or "or more."

While various aspects and embodiments have been disclosed herein, other aspects and embodiments are contemplated. The various aspects and embodiment disclosed herein are for purposes of illustration and are not intended to be limiting.

The following clauses set out aspects of the disclosure that may or may not presently be claimed:
1. A fluid collection device, comprising:
   a fluid impermeable barrier at least partially defining an interior chamber and an opening through which the interior chamber is exposed to an external environment;
   a fluid permeable body positioned at least partially within the interior chamber to extend across at least a portion of the opening and configured to wick fluid away from the opening;
   one or more retention components for retaining the fluid collection device on labia of a wearer by engaging with one or more of legs, pelvis, or buttocks of the wearer; and
   a conduit extending into the interior chamber.
2. The fluid collection device of clause 1 wherein the fluid permeable body includes a fluid permeable membrane and a fluid permeable support, wherein the fluid permeable membrane is disposed over at least a portion of a fluid permeable support.
3. The fluid collection device of any of clauses 1-2 wherein the one or more retention components include a shape of the fluid impermeable barrier and fluid permeable body, wherein the shape includes indentations in sides of the fluid impermeable barrier and fluid permeable body to accommodate surfaces of inner thighs of a wearer and wherein a front facing surface includes the opening.
4. The fluid collection device of any of clauses 1-2 wherein the one or more retention components include a pressure sensitive adhesive disposed on a front facing surface of the fluid impermeable barrier around at least a portion of the opening.
5. The fluid collection device of any of clauses 1-2 wherein the one or more retention components include one or more of straps, flaps, springs, or gripping members attached to the fluid impermeable barrier for engaging the upper legs or pelvic area to secure the device to the wearer.
6. The fluid collection device of any of clauses 1-5 wherein a front most portion of the fluid impermeable barrier and the fluid permeable body is removable from a remainder of the fluid impermeable barrier and fluid permeable body.
7. The fluid collection device of any of clauses 1-6 wherein the one or more retention components include adhesive tabs positioned on the fluid impermeable barrier to attach to thighs, buttocks, pubic region, or pelvic area of the wearer.
8. The fluid collection device of any of clauses 1-6 wherein the one or more retention components include one or more protrusions sized, shaped, and positioned on the fluid impermeable barrier to fit an anatomical feature of the wearer.
9. The fluid collection device of clause 8 wherein the one or more protrusions include a protrusion sized and shaped to fit within a gluteal cleft of the wearer.
10. The fluid collection device of any of clauses 1-2 wherein the one or more retention components include a mounting frame affixed to the fluid impermeable barrier, the mounting frame having at least semi-rigid body with a shape that at least partially complements one or more anatomical features of a wearer.
11. The fluid collection device of clause 10 wherein the mounting frame includes one or more attachment components thereon.
12. The fluid collection device of any of clauses 10-11 wherein the mounting frame includes a T-shape extending from a longitudinal axis of the fluid impermeable barrier and fluid permeable body.
13. The fluid collection device of clause 12 wherein the one or more attachment components include one or more of an adhesive, hook and loop fasteners, weights, or fasteners.
14. The fluid collection device of any of clauses 10-11 wherein the at least semi-rigid body is shaped to contour a pubic region of a wearer.
15. The fluid collection device of clause 14 wherein the mounting frame is at least partially flexible.
16. The fluid collection device of any of clauses 14-15 wherein the mounting frame includes one or more attachment components thereon.
17. The fluid collection device of any of clauses 10-16 wherein the mounting frame is configured as a spring member having a size and shape to provide inward force when the spring member is forced open.
18. The fluid collection device of clause 17 wherein the spring member includes a longitudinal member that extends along a longitudinal axis of the fluid impermeable barrier and the longitudinal member has an arcuate shape that contours a pubic region and perineal region of the wearer.
19. The fluid collection device of clause 18 wherein the spring member is disposed on or at least partially within at least a portion of the fluid impermeable barrier.
20. The fluid collection device of any of clauses 17-19 wherein the mounting frame includes a lateral member that extends substantially perpendicularly to the longitudinal axis of the fluid impermeable barrier and the lateral member has an additional arcuate shape that contours a waist region of the wearer.
21. The fluid collection device of any of clauses 17-20 wherein the spring member extends beyond a first end of the fluid impermeable barrier and a second end of the fluid impermeable barrier.
22. A fluid collection system, the system comprising:
   a fluid collection device according to any of clauses 1-21;
   a fluid storage container fluidly connected to the fluid collection device via the conduit, the fluid storage container being configured to store fluids therein; and
   a vacuum source fluidly connected to the fluid storage container, the vacuum source being configured to provide vacuum force into the interior chamber via the fluid storage container and the conduit.
23. The fluid collection system of clause 22 wherein the fluid storage container includes a canister.
24. The fluid collection system of clause 22 wherein the vacuum source includes one or more of a vacuum pump, a wall-mounted vacuum line, or a hand pump.
25. A method to collect fluid, the method comprising:
   positioning any of the fluid collection devices of clauses 1-21 adjacent to a urethra of a wearer effective to engage one or more retention components for retaining the fluid collection device with one or more of legs, pelvis, or buttocks of the wearer;
   receiving fluid from the urethra into the fluid collection device; and
   removing the fluid from the fluid collection device via the conduit.
26. The method of clause 25 wherein:
   the one or more retention components include a shape of the fluid impermeable barrier and fluid permeable body, wherein the shape includes indentations in sides of the fluid impermeable barrier and fluid permeable body to accommodate surfaces of inner thighs of the wearer and wherein a front facing surface includes the opening; and
   positioning any of the fluid collection devices of clauses 1-21 adjacent to a urethra of a wearer effective to engage one or more retention components for retaining the fluid collection device with one or more of legs, pelvis, or buttocks of the wearer includes disposing the fluid collection device between the legs of the wearer such that the indentations are disposed against the inner thighs of the wearer and the opening is disposed over the urethra.
27. The method of clause 25 wherein:
   the one or more retention components include a pressure sensitive adhesive disposed on a front facing surface of the fluid impermeable barrier around at least a portion of the opening; and
   positioning any of the fluid collection devices of clauses 1-21 adjacent to a urethra of a wearer effective to engage one or more retention components for retaining the fluid collection device with one or more of legs, pelvis, or buttocks of the wearer includes placing the pressure sensitive adhesive in contact with one or more of a pubic region, perineal region, or labia of the wearer.
28. The method of clause 25 wherein:
   the one or more retention components include one or more of straps, flaps, springs, or gripping members attached to the fluid impermeable barrier for engaging upper legs or pelvic area to secure the device to the wearer; and
   positioning any of the fluid collection devices of clauses 1-21 adjacent to a urethra of a wearer effective to engage one or more retention components for retaining the fluid collection device with one or more of legs, pelvis, or buttocks of the wearer includes engaging one or more of the straps, flaps, springs, or gripping members with one or more of the upper legs or pelvic area of the wearer.
29. The method of clause 25 wherein:
   the one or more retention components include adhesive tabs positioned on the fluid impermeable barrier to attach to thighs, buttocks, pubic region, or pelvic area of the wearer; and
   positioning any of the fluid collection devices of clauses 1-21 adjacent to a urethra of a wearer effective to engage one or more retention components for retaining the fluid collection device with one or more of legs, pelvis, or buttocks of the wearer includes placing the adhesive tabs in contact with the thighs, buttocks, pubic region, or pelvic area of the wearer.
30. The method of clause 25 wherein:
   the one or more retention components include one or more protrusions sized, shaped, and positioned on the fluid impermeable barrier to fit an anatomical feature of the wearer; and
   positioning any of the fluid collection devices of clauses 1-21 adjacent to a urethra of a wearer effective to engage one or more retention components for retaining the fluid collection device with one or more of legs, pelvis, or buttocks of the wearer includes disposing the one or more protrusions into a gluteal cleft of the wearer.
31. The method of clause 25 wherein:
   the one or more retention components include a mounting frame affixed to the fluid impermeable barrier, the mounting frame having at least semi-rigid body with a shape that at least partially complements one or more anatomical features of a wearer; and
   positioning any of the fluid collection devices of clauses 1-21 adjacent to a urethra of a wearer effective to engage one or more retention components for retaining the fluid collection device with one or more of legs, pelvis, or buttocks of the wearer includes positioning the mounting frame around the one or more anatomical features of the wearer.
32. The method of clause 31 wherein:
   the mounting frame includes one or more attachment components thereon; and
   positioning the mounting frame around the one or more anatomical features of the wearer includes attaching the one or more attachment components to the wearer or clothing of the wearer.
33. The method of clause 31 wherein:
   the mounting frame is configured as a spring member having a size and shape to provide inward force when the spring member is forced open; and
   positioning any of the fluid collection devices of clauses 1-21 adjacent to a urethra of a wearer effective to engage one or more retention components for retaining the fluid collection device with one or more of legs, pelvis, or buttocks of the wearer includes:
      opening the mounting frame;
      positioning the mounting frame around at least a portion of one or more of the legs, pelvis, or buttocks of the wearer; and
      releasing the mounting frame.
34. The method of clause 25 wherein receiving fluid from the urethra into the fluid collection devices wicking the fluid into the fluid permeable body.
35. The method of clause 25 wherein removing the fluid from the fluid collection device via the conduit includes applying a vacuum in the interior chamber.

## Claims

1. A fluid collection device (100;200;200a-c), comprising:
a fluid impermeable barrier (102; 202) at least partially defining an interior chamber (104; 204) and an opening (106;206) through which the interior chamber is exposed to an external environment;
a fluid permeable body (115;215) positioned at least partially within the interior chamber to extend across at least a portion of the opening and configured to wick fluid away from the opening;
one or more retention components (230;230a;230b;236;230c) for retaining the fluid collection device on labia of a wearer by engaging with one or more of legs, pelvis, or buttocks of the wearer,
wherein the one or more retention components include a shape of the fluid impermeable barrier and fluid permeable body, wherein the shape includes indentations (230;230a) in sides of the fluid impermeable barrier and fluid permeable body to accommodate surfaces of inner thighs of a wearer and wherein a front facing surface includes the opening; and
a conduit (108) extending into the interior chamber.

2. The fluid collection device of claim 1 wherein the fluid permeable body includes a fluid permeable membrane (118) and a fluid permeable support (120), wherein the fluid permeable membrane is disposed over at least a portion of a fluid permeable support.

3. The fluid collection device of any of claims 1-2 wherein the one or more retention components include a pressure sensitive adhesive disposed on a front facing surface of the fluid impermeable barrier around at least a portion of the opening.

4. The fluid collection device of any of claims 1-2 wherein the one or more retention components include one or more of straps, flaps, springs, or gripping members attached to the fluid impermeable barrier for engaging the upper legs or pelvic area to secure the device to the wearer.

5. The fluid collection device of any of claims 1-4 wherein a front most portion of the fluid impermeable barrier and the fluid permeable body is removable from a remainder of the fluid impermeable barrier and fluid permeable body.

6. The fluid collection device of any of claims 1-5 wherein the one or more retention components include adhesive tabs positioned on the fluid impermeable barrier to attach to thighs, buttocks, pubic region, or pelvic area of the wearer.

7. The fluid collection device of any of claims 1-5 wherein the one or more retention components include one or more protrusions (236;230c) sized, shaped, and positioned on the fluid impermeable barrier to fit an anatomical feature of the wearer.

8. The fluid collection device of claim 7 wherein the one or more protrusions include a protrusion (236;230c) sized and shaped to fit within a gluteal cleft of the wearer.

9. A fluid collection system, the system comprising:
a fluid collection device (100;200;200a-c) according to any of claims 1-6;
a fluid storage container (1719) fluidly connected to the fluid collection device via the conduit, the fluid storage container being configured to store fluids therein; and
a vacuum source (1729) fluidly connected to the fluid storage container, the vacuum source being configured to provide vacuum force into the interior chamber via the fluid storage container and the conduit.

10. The fluid collection system of claim 9 wherein the fluid storage container includes a canister.

11. The fluid collection system of claim 9 wherein the vacuum source includes one or more of a vacuum pump, a wall-mounted vacuum line, or a hand pump.

12. A method (1800) to collect fluid, the method comprising:
positioning (1810) any of the fluid collection devices (100;200;200a-c) of claims 1-8 adjacent to a urethra of a wearer effective to engage one or more retention components (230;230a;230b;236;230c) for retaining the fluid collection device with one or more of legs, pelvis, or buttocks of the wearer by disposing the fluid collection device between the legs of the wearer such that the indentations are disposed against the inner thighs of the wearer and the opening is disposed over the urethra;
receiving (1820) fluid from the urethra into the fluid collection device; and
removing (1830) the fluid from the fluid collection device via the conduit (108).

13. The method of claim 12 wherein:
the one or more retention components include one or more of straps, flaps, springs, or gripping members attached to the fluid impermeable barrier for engaging upper legs or pelvic area to secure the device to the wearer; and
positioning any of the fluid collection devices of claims 1-21 adjacent to a urethra of a wearer effective to engage one or more retention components for retaining the fluid collection device with one or more of legs, pelvis, or buttocks of the wearer includes engaging one or more of the straps, flaps, springs, or gripping members with one or more of the upper legs or pelvic area of the wearer.

14. The method of claim 12 wherein receiving fluid from the urethra into the fluid collection device includes wicking the fluid into the fluid permeable body (115;215).

15. The method of claim 12 wherein removing the fluid from the fluid collection device via the conduit (108) includes applying a vacuum in the interior chamber (104;204).
